Europäisches Patentamt

European Patent Office

Office européen des brevets

(19)

(11) Numéro de publication: **0 228 950**
**B1**

# (12) FASCICULE DE BREVET EUROPEEN

(45) Date de publication du fascicule du brevet:
**18.07.90**

(51) Int. Cl.⁵: **A61M 1/12**

(21) Numéro de dépôt: **86402747.9**

(22) Date de dépôt: **10.12.86**

(54) Module extra-péricardique pour prothèse cardiaque totale.

(30) Priorité: **13.12.85 FR 8518514**

(43) Date de publication de la demande:
**15.07.87 Bulletin 87/29**

(45) Mention de la délivrance du brevet:
**18.07.90 Bulletin 90/29**

(84) Etats contractants désignés:
**DE GB IT**

(56) Documents cités:
**EP-A- 0 146 445**
**WO-A-85/02339**
**DE-A- 3 342 534**
**US-A- 4 167 046**

(73) Titulaire: **AEROSPATIALE Société Nationale Industrielle, 37, Boulevard de Montmorency, F-75781 Paris Cédex 16(FR)**

(72) Inventeur: **Chareire, Jean-Louis, 66, rue Aristide Briand, F-92300 Levallois(FR)**
Inventeur: **Lapeyre, Didier, Chaignes, F-27120 Pacy-Sur-Eure(FR)**

(74) Mandataire: **Bonnetat, Christian et al, CABINET BONNETAT 23, Rue de Léningrad, F-75008 Paris(FR)**

## Description

Dans les demandes EP-A 0 146 445 et EP-A 0 227 537 on a décrit deux modes de réalisation d'une prothèse cardiaque totale comprenant deux pompes, respectivement représentatives du coeur droit et du coeur gauche, ainsi qu'un dispositif de commande desdites pompes, cette prothèse étant remarquable en ce que, d'une part, elle comporte l'unité fonctionnelle indissociable constituée :

- d'un module péricardique, destiné à être logé dans la cavité du coeur natural à remplacer et enfermé dans une enveloppe étanche portant au moins trois orifices de raccord destinés respectivement à être raccordés à l'oreillette droite, à l'artère pulmonaire et à l'oreillette gauche, lesdits orifices de raccord à l'oreillette droite et à l'artère pulmonaire étant pourvus de valves pour servir respectivement d'orifice d'entrée et d'orifice de sortie à une première pompe logée dans ledit module péricardique et destinée à accomplir la fonction du coeur droit du coeur naturel à remplacer;

- d'un module extra-péricardique, destiné à être logé dans un espace physiologiquement neutre du malade receveur et à accomplir la fonction du coeur gauche du coeur naturel à remplacer, ce module extra-péricardique comportant une enveloppe étanche dans laquelle est enfermée une seconde pompe pourvue d'un orifice d'entrée et d'un orifice de sortie, pourvus chacun d'une valve;

- d'une liaison fonctionnelle entre lesdits modules péricardique et extra-péricardique comportant au moins :

- un premier conduit traversant l'enveloppe dudit module péricardique et réunissant l'orifice de celui-ci correspondant à l'oreillette gauche et l'orifice d'entrée de ladite seconde pompe incorporée dans ledit module extra-péricardique;

- un second conduit établissant une communication gazeuse entre les côtés desdites première et seconde pompes opposés au sang traversant celles-ci;

et en ce que, d'autre part, ledit dispositif de commande actionne lesdites pompes en opposition.

Dans le premier mode de réalisation, l'enveloppe étanche du module péricardique comporte un quatrième orifice destiné au raccord à l'aorte et ladite liaison fonctionnelle comporte un troisième conduit traversant l'enveloppe dudit module péricardique et réunissant ledit quatrième orifice de celui-ci correspondant à l'aorte et l'orifice de sortie de ladite seconde pompe incorporée dans ledit module extra-péricardique.

En revanche, dans le second mode de réalisation, l'orifice de sortie de ladite seconde pompe incorporée dans ledit module extra-péricardique est directement raccordé à la partie abdominale de l'aorte, de sorte que l'on peut faire l'économie du quatrième orifice du module péricardique et dudit troisième conduit de liaison fonctionnelle.

Dans ces deux modes de réalisation, on réalise avantageusement les modules de pompage péricardique et extra-péricardique sous la forme de pompes à membrane et à plateau-poussoir.

La présente invention a pour objet de décrire des modes de réalisation particulièrement avantageux du module extra-péricardique pour la prothèse cardiaque totale rappelée ci-dessus.

A cette fin, selon l'invention, le module extra-péricardique pour une prothèse cardiaque totale comprenant deux pompes, respectivement représentatives du coeur droit et du coeur gauche et comportant l'unité fonctionnelle indissociable constituée :

- d'un module péricardique, destiné à être logé dans la cavité du coeur naturel à remplacer et enfermé dans une enveloppe étanche portant au moins trois orifices de raccord destinés respectivement à être raccordés à l'oreillette droite, à l'artère pulmonaire et à l'oreillette gauche, lesdits orifices de raccord à l'oreillette droite et à l'artère pulmonaire étant pourvus de valves pour servir respectivement d'orifice d'entrée et d'orifice de sortie à une première pompe logée dans ladite enveloppe et destinée à accomplir la fonction du coeur droit du coeur naturel à remplacer;

- d'un module extra-péricardique, destiné à être logé dans un espace physiologiquement neutre du malade receveur et à accomplir la fonction du coeur gauche du coeur naturel à remplacer, ce module extra-péricardique comportant une seconde pompe du type à membrane et plateau-poussoir enfermée dans une enveloppe étanche, pourvue d'un orifice d'entrée et d'un orifice de sortie, équipés chacun d'une valve;

- d'une liaison fonctionnelle entre lesdits modules comportant au moins :

- un premier conduit traversant ladite enveloppe dudit module péricardique et réunissant l'orifice de celui-ci correspondant à l'oreillette gauche et l'orifice d'entrée de la seconde pompe incorporée dans ledit module extra-péricardique;

- un second conduit établissant une communication gazeuse entre les côtés desdites première et seconde pompes opposés au sang traversant celle-ci;

et lesdites première et seconde pompes étant actionnées en opposition, est remarquable en ce que ladite seconde pompe comporte, dans son enveloppe, d'une part, une enceinte étanche en communication avec ledit orifice d'entrée et ledit orifice de sortie de ladite seconde pompe et délimitée au moins en partie par deux membranes souples en regard et, d'autre part, deux plateaux-poussoirs en regard l'un de l'autre, disposés de part et d'autre de ladite enceinte étanche au contact d'une membrane et animés de mouvements alternatifs d'éloignement et de rapprochement, sous l'action de mécanismes agencés respectivement du côté du plateau-poussoir correspondant opposé à ladite enceinte étanche.

Ainsi, en réalité, ladite seconde pompe est constituée de deux systèmes de pompages agissant sur la même enceinte étanche déformable, de sorte que, même si l'un desdits systèmes tombe en panne, le pompage peut continuer sous l'action de l'autre.

Lorsque ledit premier conduit, ainsi qu'éventuellement un conduit reliant l'orifice de sortie de ladite seconde pompe à un orifice du module péricardique relié à l'aorte, sont intérieurs audit second conduit, il est avantageux que l'enveloppe de ladite seconde pompe porte un col extérieur pour le raccord audit second conduit et un col intérieur pour le raccord de chacun desdits conduits intérieurs et que, à l'intérieur de ladite enveloppe, soient prévus des passages entre lesdits cols, pour faire comuniquer les parties de ladite enveloppe contenant lesdits mécanismes avec l'intérieur dudit second conduit.

De préférence, ledit module extra-péricardique est pourvu d'un système de raccord rapide permettant simultanément le raccord dudit second conduit et de chacun desdits conduits intérieurs avec leurs cols respectifs.

Dans un mode avantageux de réalisation, lesdites membranes sont agencées de façon à ménager entre elles au moins une gorge périphérique lorsqu'elles prennent chacune simultanément leur position de rapprochement maximal vis-à-vis de l'autre sous l'action de leurs mécanismes respectifs. Ainsi, l'enceinte étanche est parcourue par un flux sanguin permanent, favorisant le rendement de pompage et évitant les risques de thrombose.

A cet effet, chaque plateau-poussoir peut être au moins approximativement centré par rapport à la membrane correspondante et ne recouvrir que partiellement celle-ci, de façon à ménager,entre la paroi interne de l'enveloppe et sa périphérie, une portion périphérique libre de membrane. Pour favoriser encore plus la formation de ladite gorge périphérique, l'ancrage dans l'enveloppe de ladite portion périphérique de membrane souple se trouve en retrait du plan d'appui desdites membranes, lorsque celles-ci sont en position rapprochée maximale.

Afin d'améliorer encore la circulation sanguine dans ladite seconde pompe, le raccord entre ladite enceinte étanche et chacun desdits orifices d'entrée et de sortie de ladite seconde pompe se fait par l'intermédiaire d'une volute.

Par ailleurs, il est avantageux que ladite portion de membrane souple non recouverte par le plateau-poussoir correspondant soit doublée de façon étanche par au moins un anneau de matière souple. Ainsi, entre ledit anneau et ladite membrane on délimite un espace étanche dans lequel on peut disposer un capteur susceptible de détecter une détérioration de ladite membrane, dès que du sang arrive à traverser celle-ci. De même, on peut ménager aussi au moins un autre espace étanche entre cet anneau et un suivant, afin de suivre la progression d'une fuite sanguine et donc d'examiner l'état de fonctionnement du module de pompage.

Les mécanismes d'actionnement des plateaux-poussoirs peuvent être réalisés de différentes façons. Par exemple, ils peuvent être du type à vis et écrou empêché de tourner. Dans ce cas, on peut prévoir un moteur électronique à rotor et stator plats par plateau-poussoir, ledit moteur actionnant une vis axiale agissant sur un écrou solidaire dudit plateau-poussoir, qui est empêché de tourner grâce à sa solidarisation de la membrane correspondante. Les deux moteurs du module extra-péricardique peuvent avoir un mouvement de rotation alternatif et être en opposition de manière à annuler tout couple extérieur.

On peut prévoir une démultiplication entre le rotor du moteur et l'écrou lié au plateau-poussoir.

En variante, l'actionnement des plateaux-poussoirs peut être obtenu au moyen d'ensembles bielle-manivelle liés à une vis sans fin entraînée par un moteur électrique.

Les figures du dessin annexé feront bien comprendre comment l'invention peut être réalisée. Sur ces figures des références identiques désignent des éléments semblables.

La figure 1 montre schématiquement un coeur naturel en liaison avec ses veines et artères principales, en vue antérieure.

La figure 2 illustre schématiquement une prothèse du type concerné par l'invention, en place sur un patient.

La figure 3 montre schématiquement ,en vue extérieure éclatée, un premier exemple de réalisation conforme à l'invention du module de pompage extra-péricardique pour prothèse cardiaque totale du type rappelé ci-dessus, plus spécialement dans le cas où l'aorte est reliée au module péricardique.

Les figures 4,5,6 et 7 sont des vues en coupe, respectivement selon les lignes IV-IV, V-V, VI-VI et VII-VII de l'exemple de réalisation du module extra-péricardique montré sur la figure 3, représenté avec une partie de sa liaison fonctionnelle au module péricardique (non représenté) de ladite prothèse.

Les figures 8 à 12 illustrent schématiquement, en vues correspondant respectivement aux figures 3 à 7, un second exemple de réalisation selon l'invention du module de pompage extra-péricardique, plus spécialement dans le cas où l'aorte est reliée audit module extra-péricardique.

La figure 13 illustre, à plus grande échelle, le montage des plateaux-poussoirs du module extra-péricardique par rapport au boîtier de celui-ci, pour les deux modes de réalisation respectivement montrés par les groupes de figures 3 à 7 et 8 à 12.

La figure 14 montre une variante du montage de la figure 13.

Les figures 15 à 20 illustrent différents mécanismes d'actionnement pour les plateaux-poussoirs des modules extra-péricardiques montrés par les figures 3 à 12.

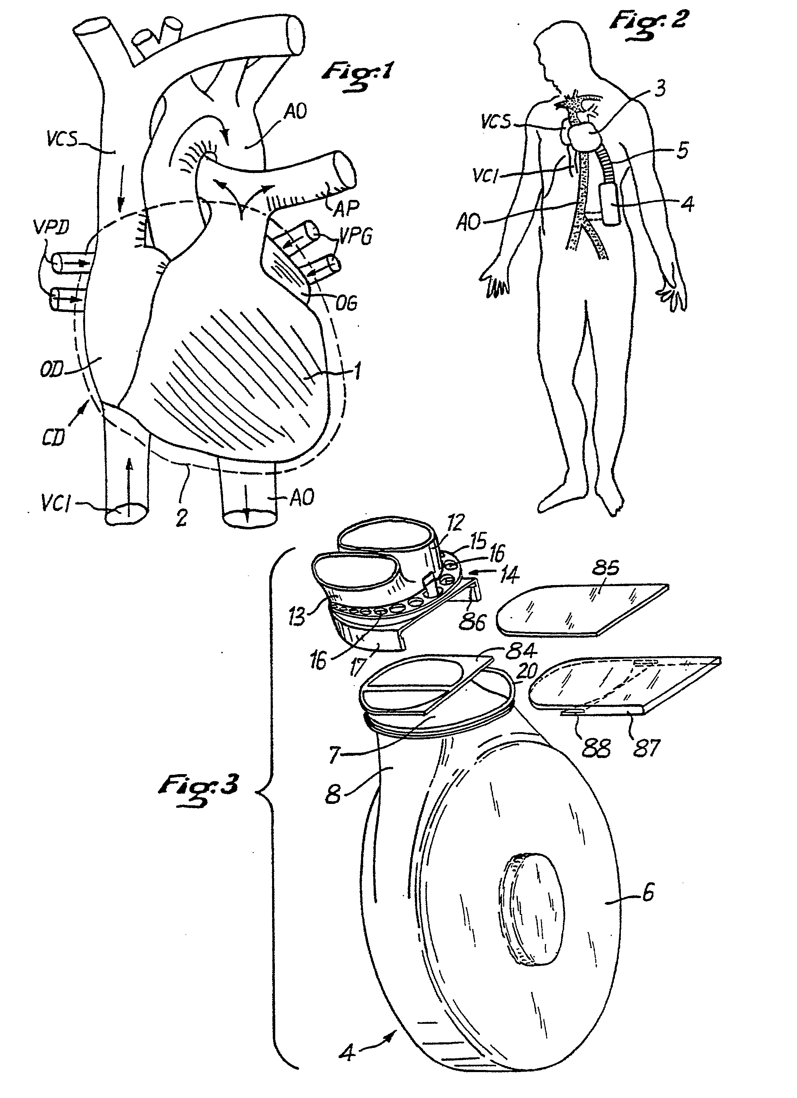

Comme illustré schématiquement sur la figure 1, un coeur humain 1 est logé dans la cavité péricardique 2 (simplement illustrée par un trait tireté 2) et est composé en réalité de deux coeurs distincts, mais solidaires l'un de l'autre, à savoir le coeur droit CD comportant l'oreillette droite OD et le ventricule droit et le coeur gauche CG comportant l'oreillette gauche OG et le ventricule gauche. L'oreillette OD du coeur droit CD reçoit le sang veineux par la veine cave supérieure VCS et par la veine cave inférieure VCI, tandis que le ventricule dudit coeur droit CD fait passer le sang ainsi reçu vers les poumons par l'intermédiaire de l'artère pulmonaire AP.

De même, l'oreillette OG du coeur gauche CG reçoit le sang provenant des poumons par les veines pulmonaires gauches VPG et droites VPD et le ventricule du coeur gauche CG chasse le sang reçu par l'aorte AO.

L'idée de base de la prothèse à pompes découplées du type auquel se rapporte la présente invention repose sur la constatation physiologique que, quoique constitué de deux pompes CD et CG formant une unité musculaire unique, le coeur 1 est en réalité composé de deux ensembles fonctionnellement indépendants. En effet, sur le plan fonctionnel, le coeur droit CD peut être considéré comme un simple coeur de passage qui pousse une colonne sanguine dont la vitesse d'écoulement est variable, mais jamais nulle, sauf lorsque les fréquences des battements du coeur 1 sont très basses. Lorsque le débit sanguin du système vasculaire augmente par suite d'une augmentation de fréquence de ces battements, la participation du coeur droit CD à la mise en mouvement du sang dans le circuit pulmonaire diminue du fait de l'augmentation de la vitesse, et donc de l'énergie cinétique, du sang parvenant au coeur droit CD. En revanche, le coeur gauche CG, par son puissant ventricule, constitue le coeur proprement dit, c'est-à-dire la pompe propulsive chargée d'assurer la perfusion sanguine de tous les organes et tissus de l'organisme.

De plus, cette idée de base s'appuie sur le fait connu de l'homme de l'art que la contraction du ventricule du coeur droit CD et celle du ventricule du coeur gauche CG peuvent être non pas simultanées, mais en opposition de phase.

Comme l'illustre très schématiquement la figure 2, cette prothèse cardiaque totale est constituée d'une unité fonctionnellement indissociable constituée de deux modules de pompage 3 et 4 découplés, mais reliés l'un à l'autre par une liaison fonctionnelle tubulaire 5.

Le module de pompage 3, destiné à remplacer le coeur droit CD du coeur naturel 1, est logé dans la cavité péricardique 2. Il comporte une enveloppe étanche sur laquelle sont montés des embouts de raccord de tout type connu, respectivement destinés à le raccorder à l'oreillette droite OD (réservoir des veines caves VCI et VCS), à l'artère pulmonaire AP, à l'oreillette gauche OG (réservoir des veines pulmonaires VPG et VPD) et éventuellement, à l'aorte AO, après coupe de celles-ci et exérèse des ventricules naturels de la cavité péricardique 2.

Les orifices de raccord à l'oreillette droite OD et à l'artère pulmonaire AP sont pourvus de valves pour servir respectivement d'orifice d'entrée et d'orifice de sortie à une pompe logée dans ladite enveloppe et destinée à accomplir la fonction du coeur droit du coeur naturel à remplacer.

Le module de pompage 4, destiné à jouer le rôle du coeur gauche CG du coeur naturel 1, est logé à l'extérieur de la cavité péricardique 2, dans un espace physiologiquement neutre, par exemple du thorax ou de l'abdomen. Il comporte également une enveloppe étanche enfermant une pompe pourvue d'un orifice d'entrée et d'un orifice de sortie, équipés chacun d'une valve.

La liaison fonctionnelle 5, qui peut sans inconvénient traverser le diaphragme du patient recevant la prothèse, comporte :
- un conduit assurant la connexion entre l'orifice du module péricardique 3 correspondant à l'oreillette gauche OG et l'orifice d'entrée de la pompe incorporée dans le module extra-péricardique 4;
- éventuellement, lorsque l'orifice de sortie de la pompe du module extra-péricardique n'est pas relié directement à l'aorte abdominale, un conduit assurant la connexion entre l'orifice du module péricardique 3 correspondant à l'aorte AO et l'orifice de sortie de ladite pompe incorporée dans ledit module extra-péricardique 4;
- un conduit établissant une communication gazeuse entre les côtés des pompes du module péricardique et du module extra-péricardique opposés au sang traversant celles-ci.

En outre, un dispositif de commande (non représenté) actionne les deux pompes en opposition et contrôle les valves disposées dans lesdits orifices.

Sur la figure 2, on a représenté schématiquement en pointillés le fait que l'orifice de sortie de la pompe du module extra-péricardique 4 pouvait être directement relié à la partie abdominale de l'aorte. Dans ce cas, le module péricardique 3 ne comporte pas d'orifice de raccord à l'aorte et la liaison 5 ne comporte que deux conduits.

Ainsi, le module extra-pericardique 4 de la prothèse totale du type rappelé ci-dessus est destiné à remplacer le coeur gauche du coeur naturel à remplacer. C'est donc lui qui effectue un travail de pompage important et supporte la fatigue la plus grande. Il est par conséquent nécessaire que son système de pompage soit particulièrement soigné, même si ledit module extra-péricardique 4 est disposé dans un espace physiologiquement neutre, tel que l'abdomen, facilement accessible par une opération chirurgicale bénigne, de sorte que son remplacement éventuel est aisé. Du fait que ce module extra-péricardique 4 se trouve dans un espace dont le volume peut être important, la réalisation du système de pompage n'a pas à respecter des contraintes d'encombrement critiques, ce qui favorise l'obtention d'une qualité élevée pour le système de pompage dudit module.

L'exemple de réalisation du module de pompage extra-péricardique 4, montré par les figures 3 à 7, est destiné au premier mode de réalisation de prothèse rappelé ci-dessus, dans lequel le module péricardique 3 est relié à l'aorte. Il comporte une enveloppe ou boîtier 6 en forme de dique, enfermant le mécanisme de pompage (non représenté sur les figures 3 à 6) et pourvu de cols emboîtés de raccordement périphérique 7 et 8, dont les axes sont radiaux par rapport à ladite enveloppe 6. Sur les cols de raccordement 7 et 8 peut se fixer un système de raccord rapide pour la liaison 5 au module péricardique 3. Ces cols 7 et 8 se raccordent latéralement aux parois intérieures périphériques du boîtier 6 par les volutes 35 et 36.

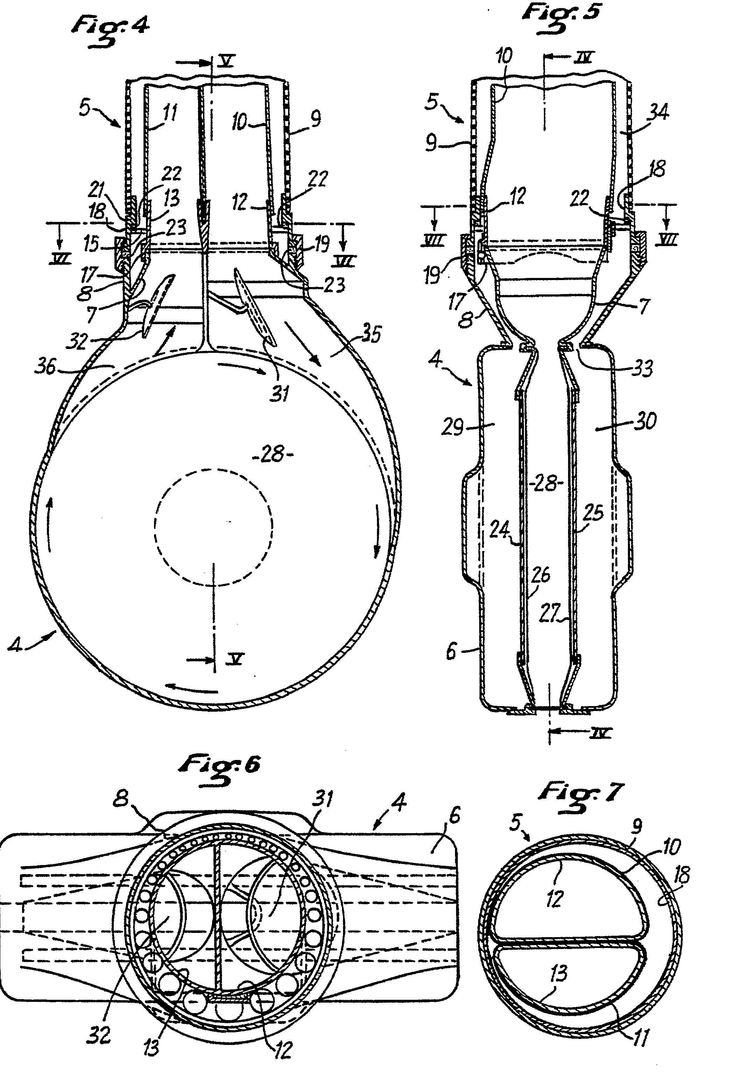

Comme on peut le voir sur les figures 4,5 et 7, cette liaison 5 comporte un tube extérieur 9, une veine pulmonaire commune artificielle 10 et le prolongement artificiel 11 de l'aorte. La veine pulmonaire 10 et l'aorte 11 sont entourées et protégées par le tube extérieur 9. La veine pulmonaire 10 est par exemple en un polyuréthane souple, comme cela a été expliqué dans les brevets rappelés ci-dessus, et joue le rôle d'oreillette gauche complémentaire, ainsi que celui d'adaptation des pressions gazeuses à la pression atmosphérique locale, puisqu'elle est en relation avec la pression d'air des poumons. Pour pouvoir remplir son office, le tube extérieur 9 est rigide radialement, mais souple en flexion et élastique longitudinalement. A cet effet, il peut être réalisé au moyen d'une spirale d'un fil rigide enrobée dans un élastomère souple.

La veine pulmonaire 10 et l'aorte 11 sont respectivement fixées sur des embouts 12 et 13 d'une pièce 14 pouvant être solidarisée de l'extrémité libre du col intérieur 7. Cette pièce 14, qui fait partie du système de raccord rapide de la liaison 5 au module extra-péricardique 4, comporte une bride 15, percée de trous 16, et une collerette 17. Les embouts 12 et 13, par exemple réalisés en titane, sont soudés à la bride 15 et à la collerette 17. Il sont avanta geusement recouverts intérieurement de polyuréthane ou de carbone déposé en phase vapeur.

Le tube extérieur 9 de la liaison 5 est fixé sur un manchon 18, duquel est prisonnier un écrou 19, pouvant coopérer avec un filetage 20 prévu à l'extrémité du col extérieur 8. Le manchon 18 comporte un épaulement 21 contre lequel s'appuie la périphérie de la bride 15, par l'intermédiaire d'un joint élastique 22. Un autre joint élastique 23 est disposé entre le manchon 18 et le filetage 20.

Ainsi, le serrage de l'écrou 19 assure simultanément la fixation du tube extérieur 9 sur le col extérieur 8 et la fixation de la veine pulmonaire artificielle 10 et de l'aorte artificielle 11 sur le col intérieur 7. En effet, en se vissant dans le sens du serrage, l'écrou 19 plaque le manchon 18 contre l'extrémité libre du col extérieur 8 et la collerette 17 de la pièce 14 contre l'extrémité libre du col intérieur 7, l'étanchéité étant assurée par les joints 22 et 23.

Le manchon 18 et l'écrou 19 peuvent être réalisés en titane ou en acier inoxydable.

A l'intérieur du boîtier 6 sont disposés deux plateaux-poussoirs 24 et 25, agissant respectivement sur des membranes 26 et 27. Les plateaux-poussoirs 24 et 25 se font face et ils sont parallèles au plan médian du boîtier 6. Ils partagent l'intérieur du boîtier 6 en une cavité intérieure de pompage 28 et en deux espaces périphériques 29 et 30 dans lesquels sont disposés les mécanismes d'actionnement (non représentés sur la figure 5) desdits plateaux-poussoirs 24 et 25. Sous l'action de ces mécanismes, les deux plateaux-poussoirs 24 et 25 peuvent se rapprocher ou s'écarter l'un de l'autre.

La cavité intérieure 28 peut être mise en communication avec la veine pulmonaire 10 et avec l'aorte 11, respectivement par l'intermédiaire de soupapes 31 et 32. Les espaces périphériques 29 et 30 sont en communication gazeuse (par les passages 33) avec le volume intérieur 34 du tube 9, non occupé par la veine pulmonaire 10 et l'aorte 11, par l'intermédiaire des trous 16 de la bride 15.

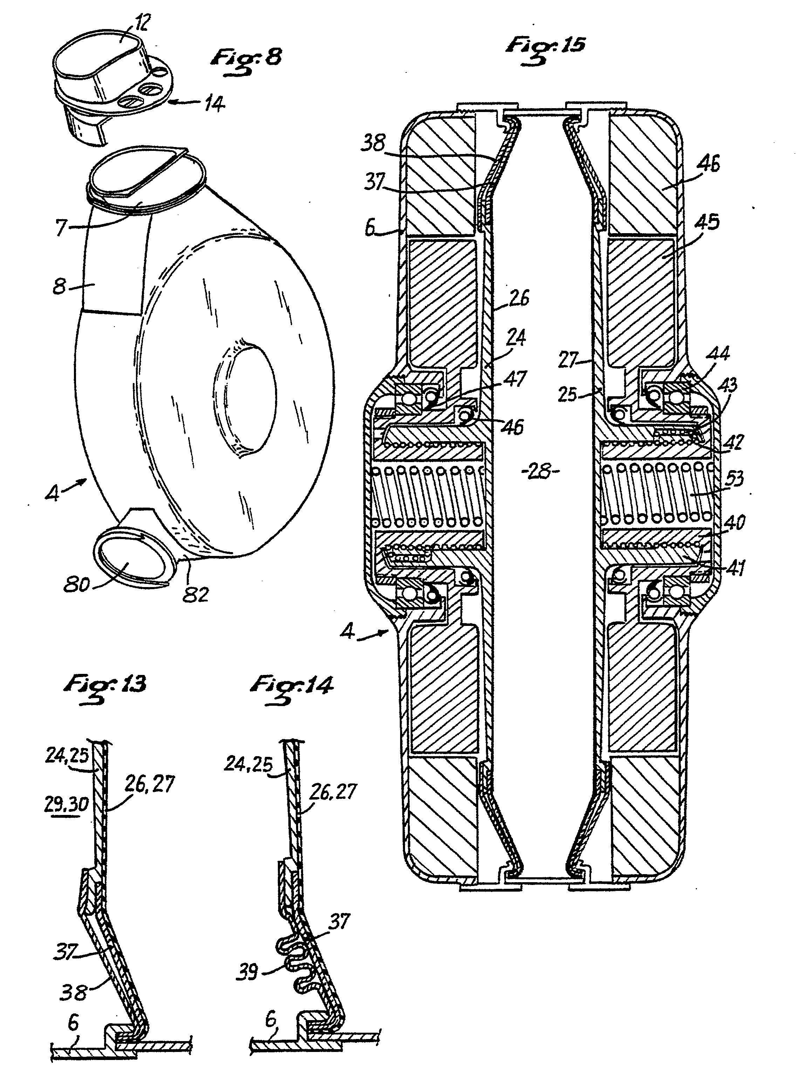

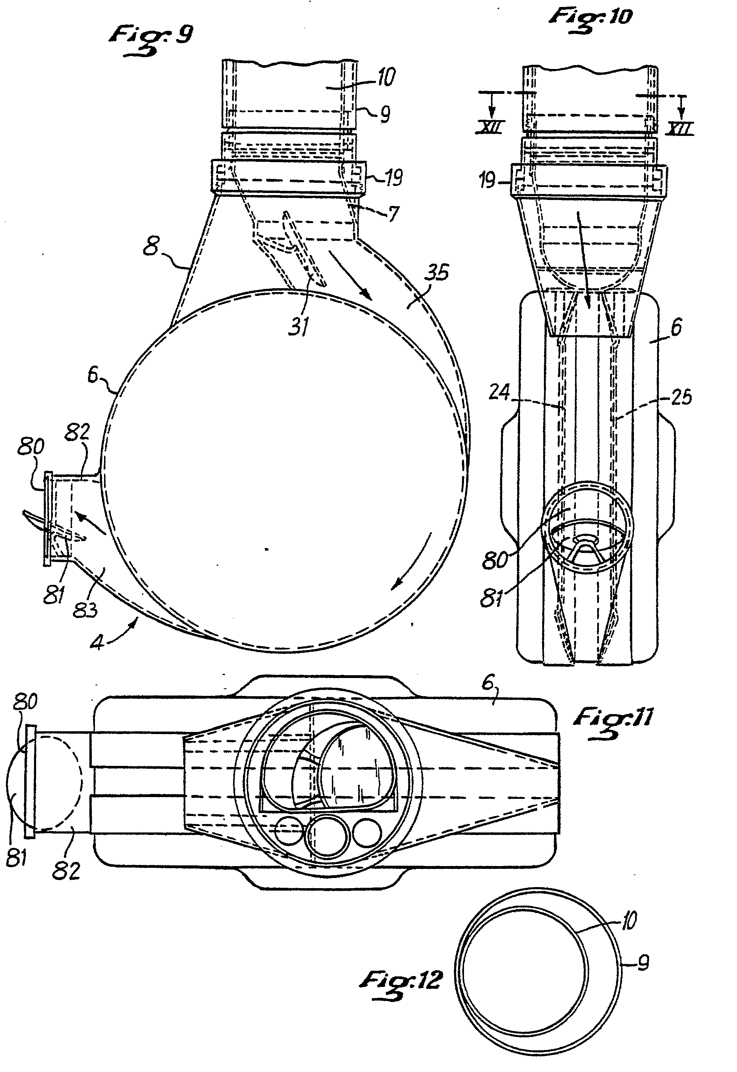

Sur les figures 8 à 12, on a représenté, respectivement en vue de l'extérieur en perspective éclatée, en vue de face, en vue de côté, en vue de dessus et selon la coupe XII-XII de la figure 10, un mode de réalisation d'un module extra-péricardique 4 selon l'invention, pour la prothèse dans laquelle ledit module est relié directement à la partie abdominale de l'aorte. Dans ce cas, l'aorte artificielle 11 est supprimée et seule le tuyau 10 se trouve dans le tube extérieur 9.

Cependant, on prévoit alors un orifice latéral 80, équipé d'une soupape 81 et d'un embout de raccord rapide 82 pour la connexion à la partie abdominale de l'aorte. Bien entendu, dans ce cas, une volute 83 peut relier l'orifice 80 à l'intérieur du boîtier 6.

Pour l'essentiel, les modules des figures 3 à 7 d'une part et des figures 8 à 12 d'autre part sont semblables et enferment les mêmes mécanismes et les mêmes dispositifs de membranes.

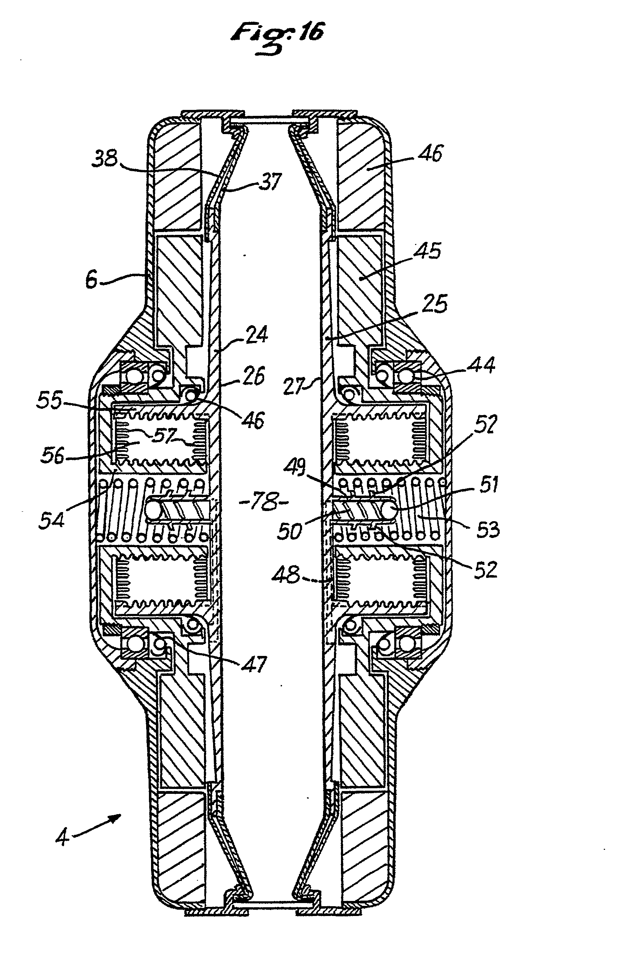

Comme on peut le voir également sur les figures 13, 14, 15, 16, 17 et 19, chacun des plateaux-poussoirs 24 et 25 est articulé à la paroi interne du boîtier 6 par l'intermédiaire d'un anneau périphérique 37 d'une matière souple inextensible, comme par exemple une toile naturelle ou synthétique enduite d'élastomère. Les anneaux 37 sont solidaires de la périphérie des plateaux-poussoirs par leur bord intérieur et solidaires du boîtier 6 par leur bord extérieur. Les lignes d'ancrage des bords extérieurs des anneaux 37 le long de la paroi interne du boîtier sont parallèles et écartées. Ainsi, même lorsque les deux plateaux-poussoirs d'un module 4 arrivent presque en appui l'un contre l'autre, les anneaux 37 correspondants ménagent entre eux une gorge périphérique.

Les deux membranes 26 et 27 sont par exemple en un polyuréthane souple afin d'être hémocompatibles. Elles recouvrent totalement les plateaux 24, 25 et les anneaux d'articulation correspondant 37, du côté de la cavité intérieure de pompage 28.

Dans le mode de réalisation de la figure 13, l'anneau d'articulation 37 est doublé par un anneau 38, disposé du côté de l'espace périphérique 29 ou 30, correspondant. Cet anneau additionnel 38 a une fonction de sécurité, comme on le verra ci-après, et permet de diminuer l'effort d'arrachement qui s'exerce à la jonction, par exemple collée, entre l'anneau 37 et le plateau 24, 25, lorsqu'il existe une pression gazeuse.

Dans la variante de réalisation de la figure 14 l'anneau additionnel 38 est remplacé par une rondelle élastique 39 pourvue de plis concentriques et pouvant être réalisée en acier, en matière synthétique, etc... Grâce à sa structure plissée, cette rondelle élastique 39 ne subit pas d'efforts radiaux importants et son rôle est à la fois la sécurité d'étanchéité, l'opposition au décollement des membranes 26, 27, ainsi que l'atténuation des contraintes que subissent les membranes et les anneaux 37 à leurs jonctions avec les plateaux 24, 25 et avec la paroi intérieure du boîtier 6.

Ainsi, lorsque les plateaux-poussoirs 24 et 25 s'écartent l'un de l'autre sous l'action de leur mécanisme d'actionnement respectifs et que la soupape d'admission 31 est ouverte (position représentée sur les figures 4 et 9), le sang provenant de la veine artificielle 10 est aspiré dans la cavité intérieure 28, dont le volume augmente. Pour pénétrer dans la cavité intérieure 28, le sang passe par la volute de répartition 35. Ensuite, lorsque les plateaux-poussoirs 24 et 25 se rapprochent l'un de l'autre et que la soupape d'échappement 32 ou 81 s'ouvre (comme représentée sur les figures 4 et 9), le sang précédemment aspiré dans la cavité intérieure 28 est expulsé dans l'aorte artificielle 11, ou vers l'aorte abdominale, en passant par la volute de sortie 36 ou 83.

Les volutes 35,36 et 83, les soupapes 31,32 et 83 et d'autres éléments de la pompe à plateaux-poussoirs peuvent être en un métal ou en alliage biocompatible, tel que le titane, l'acier inoxydable, le vitallium, etc... Ils peuvent également être en une matière non biocompatible, mais alors ils sont recouverts d'un revêtement biocompatible. Ces divers éléments sont assemblés par exemple par soudure, par microplasma, bombardement électronique ou laser.

Lors du pompage, les plateaux-poussoirs 24,25 deviennent presque jointifs, mais ils ménagent toujours entre eux un écart de quelques dixièmes de millimètre pour le sang.

La déformation en cône qu'ils subissent sous l'effort dû à la pression permet par ailleurs une meilleure évacuation radiale du sang. Enfin, il demeure toujours un volume libre torique à la périphérie des plateaux-poussoirs, de sorte que le sang peut y circuler en permanence en effectuant un mouvement tourbillonnaire continu. Le sens de circulation du sang (indiqué par les flèches sur les figures 4 et 9) est toujours le même dans la pompe, que celle-ci soit en position de remplissage ou de vidange, grâce à la disposition des volutes d'accès et sortie 35,36 et 83. Grâce à cette disposition, le rendement hydraulique de pompage est très bon et par ailleurs, le risque de thrombose est très faible,puisque toutes les parois sont activement balayées par un flux sanguin permanent. Cette disposition symétrique permet, en outre, un faible déplacement des membranes, donc une faible fatigue mécanique.

Des exemples de systèmes d'actionnement mécanique des plateaux-poussoirs 24,25 sont représentés sur les figures 15 à 20. Les plateaux, les membranes et les volutes sont semblables pour chacun des cinq exemples qui vont être décrits.

Le premier exemple (voir la figure 15) comprend, pour chaque plateau-poussoir 24 ou 25, une vis 40 et un écrou à circulation de billes 41 solidaire du plateau. La circulation des billes 42 a lieu dans la zone de l'écrou 41 qui reste toujours en prise avec la vis 40. La communication des billes entre les deux extrémités utiles de l'écrou se fait par un canal 43.

La vis 40 tourne sur son axe grâce à un roulement à billes à gorge profonde 44 qui permet un repérage géométrique suffisant. La vis 40 est enfin reliée au rotor 45 d'un moteur électrique plat dont le stator annulaire porte la référence 46.

L'obtention du déplacement axial d'un plateau 24,25 s'obtient par rotation du rotor 45 qui fait tourner la vis 40, ledit plateau étant immobilisé en rotation par les membranes 26,27,37,38,39.

La vis à circulation de billes et le roulement 44 sont lubrifiés par une huile (par exemple) qui est d'une viscosité juste suffisante pour permettre son écoulement très lent en l'absence de pression motrice. Ce lubrifiant (ne remplissant qu'une faible fraction de volume disponible) est retenu par les bagues d'étanchéité à lèvres 46 et 47.

Pour éviter que les pressions et dépressions successivement imposées au lubrifiant ne favorisent les fuites, on utilise (pour les trois systèmes à vis des figures 15, 16 et 17) le système qui est représenté uniquement sur la figure 16 à des fins de simplification de dessin.

Ce système comporte un canal 48 percé dans l'épaisseur des plateaux 24,25 et qui relie un tube 49 aux parties de la pompe non soumises aux grands écarts de pression et ne contenant (sauf accident) jamais de lubrifiant. Le tube 49 renferme un ressort 50 qui appuie sur une bille (soupape) 51.

Ainsi, lorsque le plateau 24,25 s'éloigne de la partie centrale de la pompe (phase de remplissage du sang) et que du gaz est comprimé dans le volume de la vis confiné par les joints d'étanchéité 46 et 47, ce gaz peu s'échapper facilement par le tube 49 et le conduit 48, puisque la bille 51 comprime le ressort 50.

Au contraire, lorsque le plateau 24,25 se rapproche du centre de la pompe, donc du plateau opposé , le gaz ne peut pénétrer dans le volume où se trouve la vis puisque la bille 51 fait office de soupape fermée. La dépression relative ainsi créée dans le volume où se trouve la vis favorise donc la rentrée à l'intérieur de ce volume du lubrifiant qui aurait eu tendance à franchir les bagues 46 et 47 d'étanchéité.

En pratique, la bille 51 ne crée volontairement pas une étanchéité parfaite, de sorte que ce système a surtout pour mission d'éviter toute surpression de lubrifiant de l'intérieur vers l'extérieur au niveau des joints d'étanchéité.

La bille 51 n'est jamais atteinte par le lubrifiant, quelle que soit l'orientation de la pompe car, d'une part le volume de celui-ci est faible par rapport au volume confiné par les joints 46 et 47, et d'autre part, la bille est au centre de ce volume; de plus, le corps extérieur du tube 49 comprend deux ailettes 52 et l'ensemble est revêtu d'un produit qui s'oppose à son mouillage par le lubrifiant.

Le roulement à bille 44 et le stator 46 sont fixés sur le boîtier 6, réalisé en plusieurs parties permettant le montage. Ce boîtier peut être en métal biocompatible ou revêtu d'un dépôt biocompatible ou être en matière plastique biocompatible (polycarbonate, par exemple).

Dans l'un ou l'autre cas, le système n'ayant pas besoin d'être démontable, il est fait largement appel aux assemblages par soudure laser ou bombardement électronique ou aux collages. Sur les schémas, il est volontairement représenté un certain nombre de filetages inutiles en série, mais qui favorisent la compréhension.

On comprend que le pompage du sang est obtenu grâce à une rotation d'un nombre fini de tours du rotor dans un sens, suivi d'un même nombre fini de tours dans l'autre sens.

Les réactions mécaniques extérieures à la pompe,qui pourraient être dues au mouvement alternatif des rotors, sont supprimées puisque les deux rotors du module 4 fonctionnent en opposition.

Un ressort 53, hélicoïdal, est disposé sur l'axe de chaque plateau 24,25, au centre de la vis. Son rôle est de diminuer la puissance nécessaire du moteur, car il se comprime pendant le remplissage du module (puissance de pompage quasiment nulle) et se détend pendant la vidange, en diminuant ainsi l'effort à fournir par le moteur.

Les deux moteurs électriques 45,46 du module 4 sont pilotés, d'une part, en vitesse moyenne (rythme de pompage) et, d'autre part, cycliquement pour l'obtention de la courbe de pression la plus physiologique possible dans le sang.

Le système d'actionnement illustré par la figure 16 est identique en tous points au système de la figure 15, à l'exception de la vis 54, de l'écrou 55 et des satellites 56, qui forment un roulement hélicoïdal du type "TRANSROL".

L'ensemble des pièces 54, 55 et 56 constitue en effet une vis-roulement hélicoïdal. Cette technologie est répandue, notamment dans l'aéronautique, et son but est principalement d'obtenir des durées de vie supérieures à celle des vis à billes à charge identique. Cependant, elle est utilisée ici d'une façon originale car son rôle est aussi de démultiplier le mouvement de manière à pouvoir faire tourner les moteurs plus rapidement qu'avec les vis à billes et à diminuer leur encombrement et leur masse.

Le fonctionnement de cet ensemble est le suivant:

Les n satellites filetés 56 comportent à leurs deux extrémités des dentures d'engrenages 57 qui leur permettent de rouler à l'intérieur de l'écrou 55 tout en demeurant équidistants et parallèles. Le nombre de filets des satellites 56 et de l'écrou 55 sont déterminés pour qu'il n'y ait aucun déplacement longitudinal des satellites par rapport à l'écrou. Le nombre des filets de la vis et leur sens sont déterminés pour avoir un déplacement axial faible de la vis par rapport à l'écrou pour chaque tour de vis. Le déplacement axial maximal peut ainsi nécessiter un nombre de tours moteur beaucoup plus élevé que dans le cas des vis à billes.

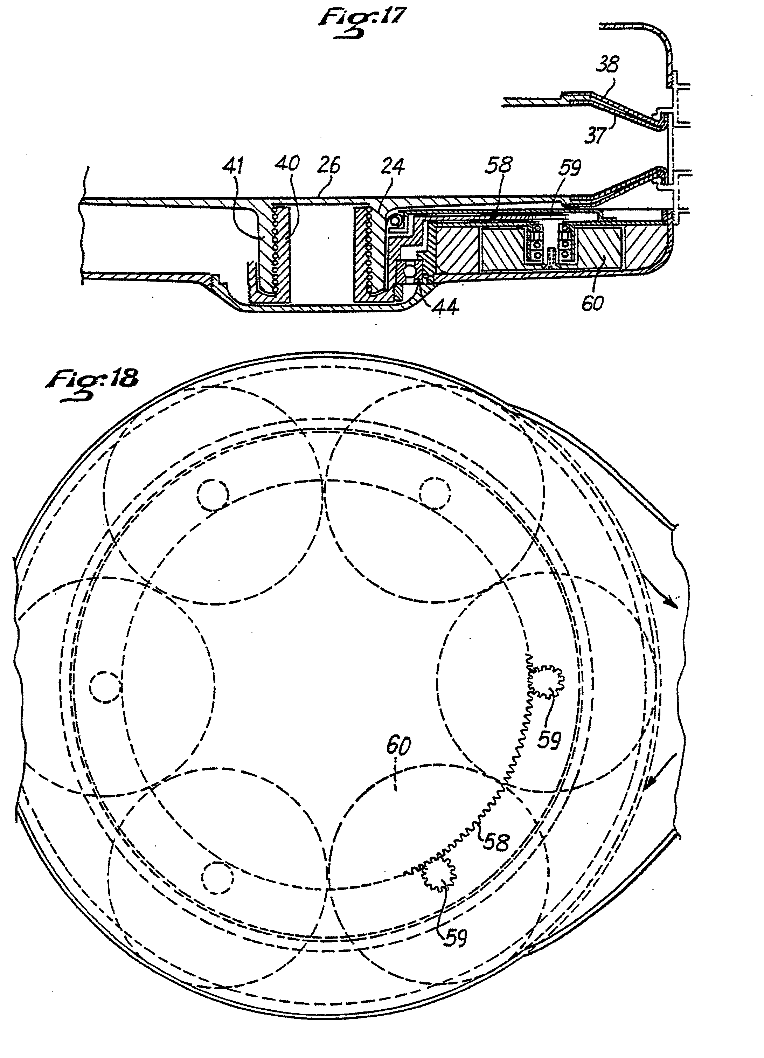

Le système d'actionnement montré schématiquement par les figures 17 et 18 est identique au système de la figure 15 en ce qui concerne la vis à billes. En revanche, celle-ci n'est pas reliée directement au rotor d'un moteur.

Elle est solidaire d'une couronne d'engrenage 58 actionnée par un certain nombre d'engrenages de très faible diamètre 59. Ceux-ci sont reliés à autant de rotors 60 de petits moteurs électriques. Cette solution permet aussi d'utiliser des moteurs à grande vitesse de rotation.

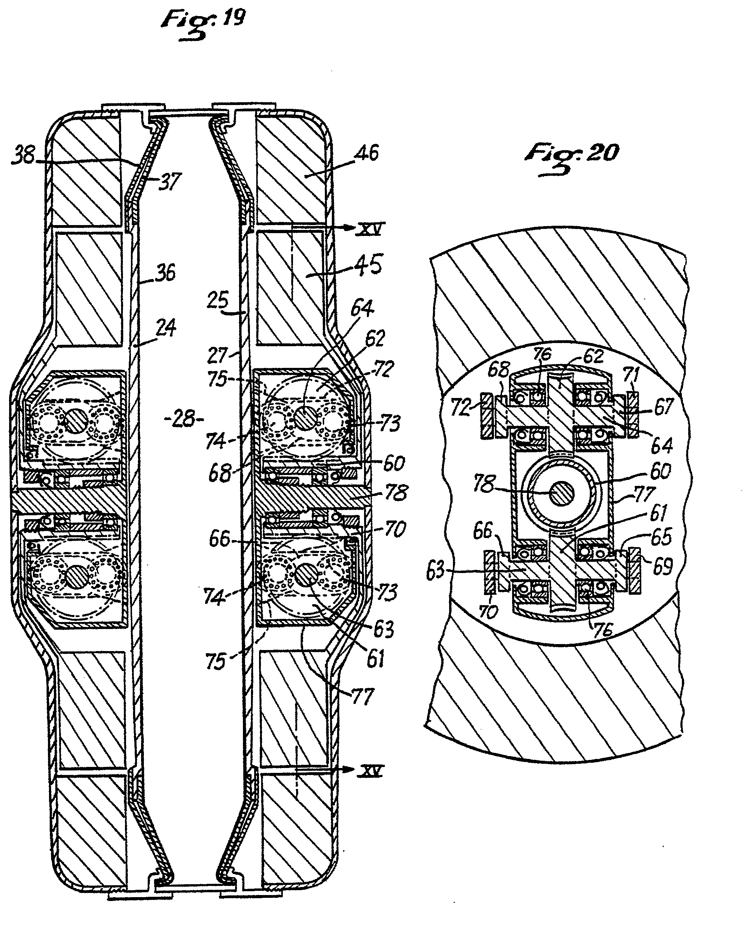

Le système d'actionnement illustré par les figures 19 et 20 diffère des trois précédents par le fait qu'il utilise deux moteurs électriques tournant toujours dans le même sens.

Le rotor 45 de chacun des deux moteurs actionne une vis sans fin 60.

Celle-ci actionne deux roues dentées 61 et 62 qui sont bloquées sur des arbres 63 et 64 respectivement.

Ceux-ci entraînent chacun deux manivelles : 65 et 66 pour l'arbre 63,67 et 68 pour l'arbre 64. Ces quatre manivelles entraînent chacune une bielle 69,70,71,72 par l'intermédiaire d'un axe muni d'un roulement à bille 73.

Enfin ces quatre bielles transmettent leur effort, par l'intermédiaire de quatre autres roulements à billes 74, à quatre axes fixés sur des supports 75 solidaires des plateaux 24,25.

L'ensemble de la vis sans fin 60, des deux roues dentées 61 et 62 et des quatre roulements à billes 76 qui maintiennent en position les arbres 63 et 64 est enfermé dans une enceinte étanche 77 qui sert également de support mécanique pour l'ensemble des pièces en mouvement. Cette enceinte 77 est par exemple en acier ou en titane et elle est soudée sur l'axe fixe 78, solidaire du boîtier 6. Tous les efforts mécaniques pour l'actionnement des plateaux 24,25 sont donc supportés par l'axe 78, mais compte tenu de la symétrie de mouvement des quatre systèmes bielle-manivelle, ces efforts sont limités à de la traction pure ou à de la compression pure avec une légère contrainte de torsion due à l'action du rotor 45.

Les quatre systèmes d'actionnement précédemment décrits présentent les avantages suivants :
- faible fatigue mécanique sur les membranes 25,26 du fait des formes et des déplacements très faibles;
- fiabilité mécanique grâce aux faibles contraintes mises en jeu, à la possibilité de prévention des pannes, à la lubrification permanente de toutes les pièces en mouvement et surtout grâce à la possibilité de mise hors service d'un des deux plateaux de pompage sans autre inconvénient que de devoir augmenter le rythme de l'autre plateau;
- fiabilité des membranes grâce à leurs dispositions respectives, à la possibilité de prévention des ruptures et à la possibilité d'arrêt d'un des moteurs en cas de détection de fuite sur la membrane concernée;
- confort pour le malade grâce à une réaction mécanique nulle des couples moteur (sauf en cas de mise hors service de l'un des deux moteurs ce qui ne correspond qu'à un service très provisoire, et grâce à des vitesses de rotation relativement faibles aboutissant à un niveau de bruit très bas;
- possibilité de mise en route progressive après l'opération du malade; celui-ci, habitué à un débit cardiaque faible, a intérêt à ne pas le voir augmenter brutalement. Grâce aux trois premiers systèmes décrits, on peut facilement augmenter progressivement le débit (indépendamment du rythme) en agissant sur la course des plateaux-poussoirs qui peut se régler à volonté;
- très bon rendement hydraulique grâce au mouvement circulaire permanent du sang et à la position des volutes d'accès et de sortie;
- très bonne résistance à la thrombose pour la raison précédente;
- facilité de pose initiale et surtout de remplacement sans obligation de placer le malade en circulation sanguine extra-corporelle;

Les préventions des pannes peuvent s'effectuer de la manière suivante :

1°) Détérioration de membranes.

En se reportant aux figures 13 et 14, on voit que pour aboutir à une fuite complète conduisant à inonder l'arrière 29,30 des plateaux 24,25 et à rendre le pompage impossible, le sang doit traverser trois épaisseurs distinctes:
- la membrane 26,27 de polyuréthane hémocompatible;
- les deux anneaux 37,38 (ou 39)

La sécurité consiste à déceler les fuites entre la membrane 26,27 et l'anneau 37 et entre l'anneau 37 et l'anneau 38 (ou 39).

La détection peut être réalisée par mesure d'hygrométrie ou de conductivité électrique du milieu (le sang étant un électrolyte), par détection d'éléments chimiques particuliers du sang, etc...

De cette façon, la détection sera réalisée bien avant la rupture franche entre les plateaux et le boîtier.

En cas de détection de fuite entre la membrane 26 ou 27 et l'anneau 37, il y aura déclenchement d'un signal d'alarme qui conduira le malade à subir un examen technique précis le plus rapidement possible.

En cas de détection de fuite entre la membrane 26 ou 27 et l'anneau 37, ainsi qu'entre l'anneau 37 et l'anneau 38 (ou 39) simultanément, il y aura déclenchement de l'arrêt du moteur concerné afin que la fuite ne puisse s'aggraver.

Si, dans un cas exceptionnel, une rupture totale de la membrane 24,25 survenait alors que les anneaux 37 et 38 (ou 39) sont déjà rompus, les conséquences seraient peu graves puisque le moteur s'arrêterait automatiquement : en effet, la circulation du sang entre les différentes membranes conduirait, en l'absence de mouvement de pompage, à une thrombose rapide du sang qui colmaterait les fuites.

L'emploi des précautions précédentes conduit donc à une garantie quasi absolue de survie du malade en cas de défection accidentelle de membrane, dans la mesure où une panne ne survient pas sur le deuxième groupe de membranes pendant le délai de remplacement de l'appareil.

2°) Pannes mécaniques

Les pannes mécaniques sérieuses telles que grippage de roulement à bille par exemple conduisent à des surintensités qui peuvent mettre le moteur concerné hors service sans nuire à la possibilité de pompage de l'appareil jusqu'à son remplacement dans un délai de quelques jours à quelques semaines.

Les pannes plus bénignes telles que (par exemple) la rupture d'une seule des quatre bielles du système des figures 19 et 20 peuvent être décelées par le microprocesseur pilotant la prothèse qui comparera en permanence le fonctionnement des deux groupes d'actionne ment des plateaux pousseurs. Toute dissymétrie relevée déclenchera l'alarme.

Par ailleurs, comme la défectuosité la plus probable est la fuite de lubrifiant, des précautions spéciales sont prises à cet effet :
- le lubrifiant est en petite quantité par rapport au volume libre gazeux total,
- il n'attaque pas chimiquement les membranes de pompage,
- il n'est pas conducteur électrique,
- il possède un marqueur chimique qui permet de déceler sa présence éventuelle dans les zones où il ne doit pas se trouver et de déclencher l'alarme.

De nombreux corps organiques ou silicones peuvent donc convenir.

On décrit maintenant ci-après le mode d'installation initial et/ou de remplacement du module extra-péricardique 4 mettant en jeu des dispositifs de purgeage de l'air dans les conduits sanguins.

On voit sur la figure 3 que la collerette 17 de la pièce 14 est destinée à recevoir la bride supérieure 84 du col 7, qui s'encastre en elle.

Le malade étant en position allongée, l'axe de la pompe est vertical et le sommet du U formé par les parois de la collerette 17 est dirigé vers le haut.

Par conséquent, comme la bride 84 s'y encastre avec précision (sur son périmètre) elle détermine entre elle et la collerette 17 un volume étanche même si elle n'est pas plaquée au fond.

Une plaque transparente 85 vient s'encastrer dans la rainure 86 et obture les conduits à sang 12 et 13.

Une autre plaque transparente 87 vient s'encastrer dans la bride 84 grâce à sa rainure 88. La surface de la bride 84 est de surcroît revêtue par un corps légèrement adhésif (graisse épaisse par exemple) permettant le glissement de la plaque 87 mais tendant à empêcher son soulèvement.

On voit donc que lorsque les plaques 85 et 87 sont en place, il est toujours possible d'encastrer la bride 84 dans la collerette 17 et si les plaques 85 et 87 viennent au contact l'une de l'autre, il n'y a plus d'air contenu dans l'intervalle en U limité par les pièces 17 et 84. Par conséquent, si la pompe à sang et les tubes 12 et 13 sont respectivement remplis de sang et obturés par les plaques 85 et 87 (la transparence de celles-ci permettant de vérifier l'absence de bulles d'air), le fait de soulever verticalement les plaques 85 et 87 ne peut pas introduire d'air dans le circuit à sang. Il suffit ensuite de rapprocher la bride 84 de la collerette 17 d'une épaisseur égale à la somme des épaisseurs des plaques 85 et 87.

## Revendications

1 - Module extra-péricardique pour une prothèse cardiaque totale comprenant deux pompes, respectivement représentatives du coeur droit et du coeur gauche et comportant l'unité fonctionnelle indissociable constituée :
- d'un module péricardique (3) destiné à être logé dans la cavité (2) du coeur naturel à remplacer et enfermé dans une enveloppe étanche portant au moins trois orifices de raccord destinés respectivement à être raccordés à l'oreillette droite OD, à l'artère pulmonaire AP et à l'oreillette gauche OG, lesdits orifices de raccord à l'oreillette droite OD et à l'artère pulmonaire AP étant pourvus de valves pour servir respectivement d'orifice d'entrée et d'orifice de sortie à une première pompe logée dans ladite enveloppe et destinée à accomplir la fonction du coeur droit du coeur naturel à remplacer ;
- d'un module extra-péricardique (4), destiné à être logé dans un espace physiologiquement neutre du malade receveur et à accomplir la fonction du coeur gauche du coeur naturel (1) à remplacer, ce module extra-péricardique comportant une seconde pompe du type à membrane et à plateau-poussoir enfermée dans une enveloppe étanche (6) pourvue d'un orifice d'entrée (35) et d'un orifice de sortie (36 ou 80) équipés chacun d'une valve ;
- d'une liaison fonctionnelle (5) entre lesdits modules comportant au moins :
- un premier conduit (10) traversant ladite enveloppe dudit module péricardique (3) et réunissant l'orifice de celui-ci correspondant à l'oreillette gauche OG et l'orifice d'entrée de la seconde pompe incorporée dans ledit module extra-péricardique (4) ;
- un second conduit (9) établissant une communication gazeuse entre les côtés desdites première et seconde pompes opposés au sang traversant celles-ci;
et lesdites première et seconde pompes étant actionnées en opposition,
caractérisé en ce que ladite seconde pompe comporte, dans son enveloppe (6) d'une part, une enceinte étanche (28) en communication avec ledit orifice d'entrée (35) et ledit orifice de sortie (36,80) de ladite seconde pompe délimitée au moins en partie par deux membranes souples en regard (26,27) et, d'autre part, deux plateaux-poussoirs (24,25) en regard l'un de l'autre, disposés de part et d'autre de ladite enceinte étanche au contact d'une membrane et animés de mouvements alternatifs d'éloignement et de rapprochement, sous l'action de deux mécanismes indépendant agencés respectivement du côté (29 ou 30) du plateau-poussoir correspondant opposé à ladite enceinte étanche.

2 - Module extra-péricardique selon la revendication 1, dans lequel ledit premier conduit (10), ainsi qu'éventuellement un conduit (11) reliant l'orifice de sortie de ladite seconde pompe à une orifice du module péricardique relié à l'aorte, sont intérieurs audit second conduit (9),
caractérisé en ce que l'enveloppe (6) de ladite seconde pompe porte un col extérieur (8) pour le raccord audit second conduit et un col intérieur (7) pour le raccord de chacun desdits conduits intérieurs et en ce que, à l'intérieur de ladite enveloppe, sont prévus des passages (16, 33) entre lesdits cols pour faire communiquer les parties (29,30) de ladite enveloppe (6) contenant lesdits mécanismes avec l'intérieur (34) dudit second conduit (9).

3 - Module extra-péricardique selon la revendication 2,
caractérisé en ce qu'il est pourvu d'un système de raccord rapide (14 à 23) permettant simultanément le raccord dudit second conduit (9) et de chacun des conduits intérieurs avec leurs cols respectifs.

4 - Module extra-péricardique selon l'une des revendications 1 à 3,
caractérisé en ce que lesdites membranes (26, 27) sont agencées de façon à ménager entre elles au moins une gorge périphérique lorsqu'elles prennent chacune simultanément leur position de rapprochement maximal vis-à-vis de l'autre sous l'action de leurs mécanismes respectifs.

5 - Module extra-péricardique selon la revendication 4,
caractérisé en ce que chaque plateau-poussoir (24,25) est au moins approximativement centré par rapport à la membrane (26,27) correspondante et ne recouvre que partiellement celle-ci, de façon à ménager entre la paroi interne de l'enveloppe et sa périphérie, une portion périphérique libre de membrane.

6 - Module extra-péricardique selon la revendication 5,
caractérisé en ce que l'ancrage dans l'enveloppe (6) de ladite portion périphérique de membrane souple (26,27) se trouve en retrait du plan d'appui desdites membranes, lorsque celles-ci sont en position rapprochée maximale.

7 - Module extra-péricardique selon l'une quelconque des revendications 1 à 6,
caractérisé en ce que le raccord entre ladite enceinte étanche (28) et chacun desdits orifices d'entrée et de sortie de ladite seconde pompe se fait par l'intermédiaire d'une volut (35, 36 ou 83).

8- Module extra-péricardique selon l'une quelconque des revendications 5 ou 6,
caractérisé en ce que ladite portion périphérique de membrane souple non recouverte par le plateau-poussoir correspondant est doublée de façon étanche par au moins un anneau de matière également souple (37, 38).

9 - Module extra-péricardique selon l'une quelconque des revendications 1 à 8,
caractérisé en ce que lesdits mécanismes d'actionnement des plateaux-poussoirs (24, 25) sont du type à vis et écrou bloqué en rotation.

10 - Module extra-péricardique selon l'une quelconque des revendications 1 à 8,
caractérisé en ce que lesdits mécanismes d'actionnement des plateaux-poussoirs (24, 25) sont du type à biellettes (66 à 70).

**Claims**

1. Extra-pericardial module for a total cardial prosthesis comprising two pumps, respectively representative of the right heart and the left heart and comprising the indissociable functional unit constituted of:
- a pericardial module (3) adapted to be housed in the cavity (2) of the natural heart to be replaced and enclosed in a tight envelope comprising at least three orifices for connection, adapted respectively to be connected to the right atrium OD, to the pulmonary artery AP, and to the left atrium OG, said orifice for connection to the right atrium OD and to the pulmonary artery AP being provided with valves in order to serve respectively as input orifice and as output orifice for a first pump housed in said envelope and adapted to perform the function of the right heart of the natural heart to be replaced;
- an extra-pericardial module (4), adapted to be housed in a physiologically neutral space in the recipient patient and to perform the function of the left heart of the natural heart (1) to be replaced, said extra-pericardial module comprising a second pump of the type with diaphragm and thrust plate enclosed in a tight envelope (6), provided with an input orifice (35) and an output orifice (36 or 80), each equipped with a valve;
- a functional link (5) between said modules comprising at least:
- a first duct (10) passing through said envelope of said pericardial module (3) and connecting the orifice thereof corresponding to the left atrium OG and the input orifice of the second pump incorporated in said extra-pericardial module (4);
- a second duct (9) establishing a gaseous communication between the sides of said first and second pumps opposite the blood passing therethrough;

and said first and second pumps being actuated in opposition, characterized in that said second pump comprises in its envelope (6), on the one hand, a tight enclosure (28) which communicates with said input orifice (35) and said output orifice (36, 80) of said second pump, and which is at least partly defined by two supple diaphragms (26, 27), placed in facing relationship, and on the other hand, two thrust plates (24, 25) also in facing relationship, placed on either side of said tight enclosure in contact with a diaphragm and imparted with reciprocating back and forth movements, under the action of two independent mechanisms disposed respectively on the side (29 or 30) of the corresponding thrust plate opposite said tight enclosure.

2. Extra-pericardial module according to claim 1, in which said first duct (10), and optionally a duct (11) connecting the output orifice of said second pump and an orifice of the pericardial module connected to the aorta, are internal to said second duct (9), characterized in that the envelope (6) of said second pump is provided with an external neck (8) for connection to said second duct, and an internal neck (7) for connection of each of said internal ducts, and in that passages (16, 33) are provided, inside said envelope, between said necks, so that the parts (29, 30) of said envelope (6) containing said mechanisms can communicate with the inside (34) of said second duct (9).

3. Extra-pericardial module according to claim 2, characterized in that said module is provided with a quick connection system (14 to 23) for simultaneous connection of said second duct (9) and of each of said internal ducts with their respective necks.

4. Extra-pericardial module according to one of claims 1 to 3, characterized in that said diaphragms (26, 27) are so arranged as to provide between them at least one peripheral channel when each one simultaneously takes its maximum close-up position with respect to the other under the action of their respective mechanisms.

5. Extra-pericardial module according to claim 4, characterized in that each thrust plate (24, 25) is at least approximately centered with respect to the corresponding diaphragm (26, 27) and covers the latter only partly, thus leaving a free peripheral portion of diaphragm between the inner wall of the envelope and its periphery.

6. Extra-pericardial module according to claim 5, characterized in that the anchoring into the envelope (6) of said peripheral portion of supple diaphragm (26, 27) is offset from the plane supporting said diaphragms, when the latter are in maximum close-up position.

7. Extra-pericardial module according to any one of claims 1 to 6, characterized in that the connection between said tight enclosure (28) and each of said input and output orifices of said second pump is achieved by way of a shell (35, 36 or 83).

8. Extra-pericardial module according to any one of claims 5 or 6, characterized in that said peripheral portion of supple diaphragm non-covered over by the corresponding thrust plate is tightly lined by at least one ring (37, 38), also in supple material.

9. Extra-pericardial module according to any one of claims 1 to 8, characterized in that said mechanisms for actuating the thrust plates (24, 25) are of the screw and nut type, locked in rotation.

10. Extra-pericardial module according to any one of claims 1 to 8, characterized in that said mechanisms for actuating the thrust plates (24, 25) are of the connecting rod type (66 to 70).

**Patentansprüche**

1. Extraperikardiales Modul für eine Gesamt-Herzprothese bestehend aus zwei jeweils das linke und das rechte Herz darstellenden Pumpen und einer nicht abzutrennenden Funktionseinheit, die

– einen Perikardmodul (3), der als Ersatz des eigentlichen Herzens in den Hohlraum (2) einzusetzen und in eine dichte Umhüllung einzuschließen ist, auf der zumindest drei Anschlußöffnungen liegen, die zu verbinden sind mit dem rechten Vorhof OD, der Pulmonalarterie AP bzw. dem linken Vorhof OB, wobei die Anschlußöffnungen mit dem rechten Vorhof OD und der Pulmonalarterie AP mit Ventilen versehen sind, um einer in der Umhüllung eingesetzten ersten Pumpe als Eingangs- bzw. Ausgangsöffnung zu dienen und die Funktion des rechten, zu ersetzenden natürlichen Herzens zu übernehmen,

– einen Extraperikardmodul (4), der in einen physiologisch neutralen Bereich des kranken Empfängers einzusetzen ist sowie die Funktion des linken, zu ersetzenden natürlichen Herzens (1) zu übernehmen hat und der eine zweite Membran- und Stangenstößel-Pumpe besitzt, die in einer dichten Umhüllung (6) eingeschlossen ist, die eine jeweils mit einem Ventil versehene Eingangs- und Ausgangöffnung (35 bzw. 36 oder 80) hat,

– und eine funktionelle Verbindung (5) zwischen, den Modulen aufweist, bestehend zumindest aus:

– einer ersten Leitung (10), die die Umhüllung des Perikardmoduls (3) durchquert sowie mit dessen Öffnung verbunden ist, die dem linken Vorhof OG und der Eingangsöffnung der zweiten, im Extraperikardmodul (4) eingebauten Pumpe entspricht, und

– einer zweiten Leitung (9), durch die eine gasführende Verbindung zwischen den Seiten der ersten und zweiten Pumpe entgegen des sie durchlaufenden Blutes hergestellt wird, wobei der Betrieb der ersten und der zweiten Pumpe gegenläufig ist,

dadurch gekennzeichnet, daß die zweite Pumpe in ihrer Umhüllung (6) einerseits eine dichte Einfassung (28), die mit der Eingangsöffnung (35) sowie der Ausgangsöffnung (36, 80) der zweiten Pumpe, die zumindest zum Teil durch die biegsamen gegenüberliegenden Membranen (26, 27) begrenzt ist, in Verbindung steht, und andererseits zwei einander gegenüberliegende Stößelstangen (24, 25) aufweist, die beiderseits der dichten Einfassung in Kontakt mit einer Membran angeordnet sind und unter der Einwirkung von zwei unabhängigen Mechanismen, die von jeweils der Seite (29 oder 30) der entsprechenden, der dichten Einfassung gegenüberliegenden Stößelstange angetrieben werden: zu wechselnden Hin- und Herführbewegungen veranlaßt werden.

2. Extraperikardmodul nach Anspruch 1, in dem die erste Leitung (10) sowie gegebenenfalls eine Leitung (11), durch die die Ausgangsöffnnng der zweiten Pumpe mit der Öffnung des an der Aorta liegenden Perikardmoduls verbunden wird, innerhalb der zweiten Leitung (9) liegen, dadurch gekennzeichnet, daß die Umhüllung (6) der zweiten Pumpe einen äußeren Kragen (8) für den Anschluß an die zweite Leitung und einen inneren Kragen (7) für den Anschluß jeder der inneren Leitungen trägt und daß im Innern der Umhüllung Durchgänge (16, 33) zwischen den Kragen vorgesehen sind, um die Teile (29, 30) der die Mechanismen enthaltenden Umhüllung (6) mit dem Innern (34) der zweiten Leitung (9) in Verbindung treten su lassen.

3. Extraperikardmodul nach Anspruch 2, dadurch gekennzeichnet, daß ein Schnellanschlußsystem (14 bis 23) vorgesehen ist, durch das gleichzeitig der Anschluß der zweiten Leitung (9) und jeder der inneren Leitungen mit den jeweiligen Kragen ermöglicht wird.

4. Extraperikardmodul nach einem der vorhergehenden Ansprüche 1 bis 3, dadurch gekennzeichnet, daß die Membranen (26, 27) derart betätigt werden, daß sie zwischen sich zumindest eine periphere Kehlung schaffen, wenn jede von ihnen gleichzeitig unter der Einwirkung ihrer jeweiligen Mechanismen ihre der anderen gegenüberliegende maximale Hinführungs- oder Annäherungsstellung einnimmt.

5. Extraperikardmodul nach Anspruch 4, dadurch gekennzeichnet, daß jede Stößelstange (24, 25) in Bezug zur entsprechenden Membran (26, 27) zumindest annähernd zentriert ist und diese nur zum Teil derart abdeckt, daß zwischen der Innenwand der Umhüllung und ihrer Peripherie ein freier peripherer Membranabschnitt geschaffen wird.

6. Extraperikardmodul nach Anspruch 5 gekennzeichnet, daß die Verankerung in der Umhüllung (6) des peripheren Abschnitts der biegsamen Membran (26, 27) von der Anlageebene der Membranen zurückversetzt liegt, wenn diese sich in der Stellung der maximalen Hinführung oder Annäherung befinden.

7. Extraperikardmodul nach einem der vorhergehenden Ansprüche 1 bis 6, dadurch gekennzeichnet, daß der Anschluß zwischen der dichten Einfassung (28) und jeder Eingangs- und Ausgangsöffnung der zweiten Pumpe über einen Spiraldiffusor (35, 36 oder 89) verläuft.

8. Extraperikardmodul nach einem der vorhergehenden Ansprüche 5 oder 6, dadurch gekennzeichnet, daß der periphere Abschnitt der biegsamen Membran, der nicht von der entsprechenden Stößelstange abgedeckt ist, durch zumindest einen Ring (37, 38) aus gleichermaßen nachgiebigen Werkstoff abdichtend verstärkt wird.

9. Extraperikardmodul nach einem der vorhergehenden Ansprüche 1 bis 8, dadurch gekennzeichnet, daß die Betätigungsmechanismen der Stößelstangen (24, 25) als drehgesperrte Schraube und Mutter ausgebildet sind.

10. Extraperikardmodul nach einem der vorhergehenden Ansprüche 1 bis 8, dadurch gekennzeichnet, daß die Betätigungsmechanismen der Stößelstangen (24, 25) als Treibstängchen oder kleiner Schwingarm (66 bis 70) ausgebildet sind.

*Fig:1*

VCS

AO

VPD

AP

VPG

OG

OD

CD

VCI

2

AO

*Fig:2*

VCS

3

VCI

5

AO

4

*Fig:3*

12

15 16

14

13

86

16 17

16

84

20

7

88 87

8

6

4

85

Fig. 4

Fig. 5

Fig. 6

Fig. 7

EP 0 228 950 B1

*Fig. 8*

*Fig. 15*

*Fig. 13*  *Fig. 14*

*Fig:9*

*Fig:10*

*Fig:11*

*Fig:12*

## Fig. 16

Fig.17

Fig.18

Fig. 19

Fig. 20